# EUROPEAN PATENT APPLICATION

(11) **EP 3 733 072 A1**
(43) Date of publication of application: **04.11.2020**
(21) Application number: 19747347.3
(22) Date of filing: 29.01.2019
(51) Int. Cl.: A61B 6/02, A61B 6/00

(54) **CONTROL METHOD OF X-RAY IMAGING DEVICE**

(30) Priority: 31.01.2018 US 201862624426 P
(71) Applicant: Nanox Imaging Ltd., 50 Town Range (GI); Nanox Imaging, Inc., Tokyo 100-0004 (JP)
(72) Inventor: KENMOTSU, Hidenori, Machida City, Tokyo 195-0055 (JP); MASUYA, Hitoshi, Kashiwa City, Chiba 277-0835 (JP); TSUCHIYA, Tadayoshi, Ishioka City Ibaraki 315-0035 (JP); NISHIMOTO, Norihito, Nagoya City, Aichi 453-0014 (JP)
(74) Representative: Kurig, Thomas
(86) International application number: PCT/JP2019/002968
(87) International publication number: WO 2019/151249

(57) **Abstract**

The object of the present invention to reduce the number of X-ray tubes constituting the distributed X-ray source. Disclosed herein is a control method for an X-ray imaging apparatus provided with a plurality of X-ray tubes arranged at fixed relative positions. The control method includes a driving step S1 of sequentially driving the plurality of X-ray tubes, and a movement step S3 of moving the plurality of X-ray tubes. The driving step S1 is executed again after execution of the movement step S3.

## Description

### [Technical Field]

The present invention relates to a control method for an X-ray imaging apparatus.

### [Background Art]

There is known an X-ray imaging apparatus that executes tomosynthesis imaging for obtaining patient tomographic images. In the X-ray imaging apparatus of this type, imaging is performed plural times while moving an X-ray tube (see, for example, JP 2017-164426 A).

### [Patent Document]

[Patent Document 1] JP 2017-164426 A

The imaging while moving an X-ray tube takes time. To cope with this, the present inventor considered that reduction of the imaging time can be achieved by providing a plurality of X-ray tubes in advance and performing imaging while sequentially switching the X-ray tubes from one to another by a control signal. The present inventors call this method "distributed X-ray source".

However, when the distributed X-ray source is used, 10 or more X-ray tubes need to be provided beforehand. When the number of X-ray tubes is increased, the price of the X-ray imaging apparatus is correspondingly increased. Further, it becomes difficult to maintain uniformity among X-ray sources, and thus, improvement is required.

It is therefore an object of the present invention to provide an X-ray imaging apparatus control method capable of achieving reduction in the number of X-ray tubes constituting the distributed X-ray source.

### [Means for Solving Problem]

An X-ray imaging apparatus control method according to the present invention is a control method for an X-ray imaging apparatus provided with a plurality of X-ray tubes arranged at fixed relative positions and includes: a driving step of sequentially driving the plurality of X-ray tubes; and a movement step of moving the plurality of X-ray tubes. The driving step is executed again after execution of the movement step.

### [Advantageous Effects of Invention]

According to the present invention, it is enough to prepare the X-ray tubes of half or less than the number of required imaging positions and, in addition, imaging time can be shortened as compared to a case when imaging is performed while moving one X-ray tube, so that it is possible to reduce the number of the X-ray tubes constituting the distributed X-ray source.

### [Brief Description of Drawings]

FIG. 1A is a view illustrating the configuration of an X-ray imaging apparatus 1 according to an embodiment of the present invention. FIG. 1B is a schematic cross-sectional view of the electron emission part 10 provided inside each of the cold cathode X-ray tubes 3.
FIGs. 2A and 2B are views explaining a distributed X-ray source G according to an embodiment of the present invention.
FIG. 3 is a flow diagram illustrating a control method for the X-ray imaging apparatus 1 according to an embodiment of the present invention.
FIG. 4 is a view explaining the control method for the X-ray imaging apparatus 1 according to an embodiment of the present invention.
FIG. 5 is a view explaining the control method for the X-ray imaging apparatus 1 according to a first modification of the embodiment of the present invention.
FIG. 6 is a view explaining the distributed X-ray source G according to a second modification of the embodiment of the present invention.

### [Mode for Carrying out the Invention]

Preferred embodiments of the present invention will be explained below in detail with reference to the accompanying drawings.

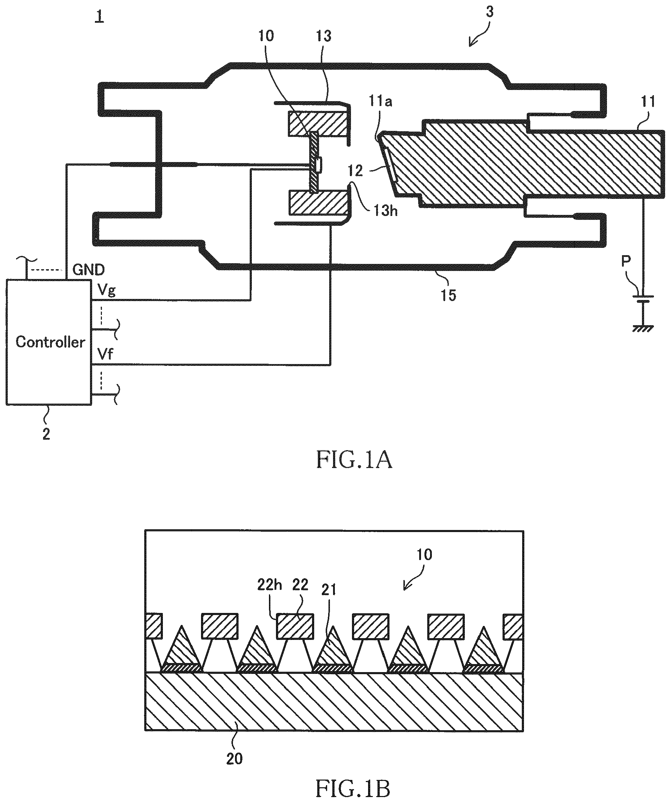

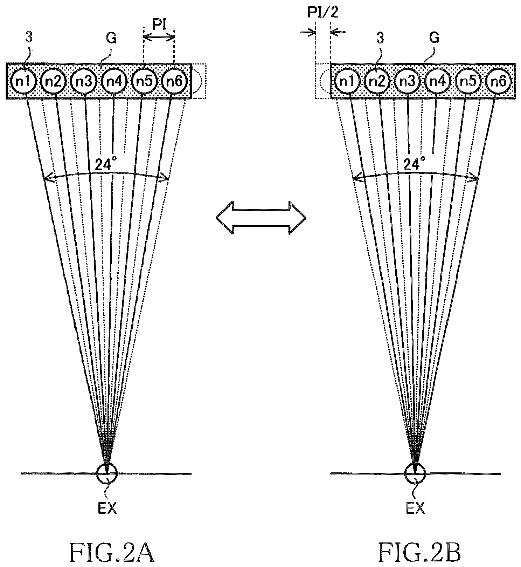

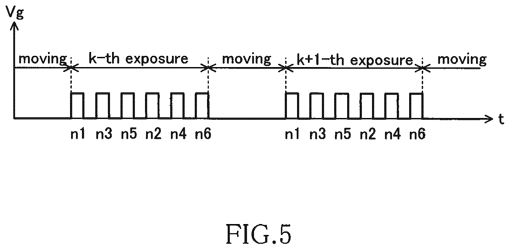

FIG. 1A is a view illustrating the configuration of an X-ray imaging apparatus 1 according to an embodiment of the present invention. The X-ray imaging apparatus 1 includes a plurality of cold cathode X-ray tubes 3 and a controller 2 for the X-ray tubes 3. FIG. 1A only illustrates the cross-section of one of the X-ray tubes 3 and the controller 2. The plurality of X-ray tubes 3 constitute a distributed X-ray source and are arranged at fixed relative positions as illustrated in FIGs. 2 and 5 to be described later.

As illustrated in FIG. 1A, each X-ray tube 3 has a structure in which an electron emission part 10, an anode part 11, a target 12, and a focus structure 13 are disposed inside a housing 15.

The housing 15 is a sealed member made of glass, ceramic, or stainless. Although not illustrated, a valve is provided in the housing 15, and exhaust of gas from the housing 15 and injection of gas into the housing 15 are performed as needed through the valve. For example, before the X-ray tube 3 is operated under the control of the controller 2, a vacuum pump is used to exhaust the gas from the housing 15 to bring the housing 15 into a vacuum state.

FIG. 1B is a schematic cross-sectional view of the electron emission part 10. As illustrated, the electron emission part 10 includes a cathode part 20, a plurality of electron emission elements 21 disposed on the upper surface of the cathode part 20, and a gate electrode 22 having a plurality of matrix-arranged openings 22h. Each of the plurality of electron emission elements 21 is a Spindt-type cold cathode and disposed in its corresponding opening 22h. The upper end of each of the electron emission elements 21 is positioned within the corresponding opening 22h. The cathode part 20 is supplied with a ground potential GND from the controller 2, and the gate electrode 22 is supplied with gate voltage Vg from the controller 2.

The anode part 11 is a metal member having an anode surface 11a disposed opposite to the electron emission part 10 and is made of copper (Cu). The anode part 11 is connected with the positive side terminal of a power supply P. Thus, when the gate electrode 22 illustrated in FIG. 1B is turned ON, current (anode current) flows from the power supply P through the anode part 11, electron emission part 10, and cathode part 20. At this time, a plurality of electrons are emitted from the electron emission elements 21 illustrated in FIG. 1B. These electrons collide with the anode surface 11a, pass through the anode part 11, and are absorbed by the power supply P. As illustrated in FIG. 1A, the anode surface 11a is inclined to the electron moving direction (direction from the left to the right in FIG. 1A).

The target 12 is a member that is made of a material that generates an X-ray by receiving electrons and disposed so as to cover a part of the anode surface 11a with which the electrons emitted from the electron emission elements 21 directly collide. Since the target 12 is disposed on the anode surface 11a, some or all of the plurality of electrons that collide with the anode surface 11a pass through the target 12, and an X-ray is generated in the target 12 during the passage. The thus generated X-ray is radiated downward in the drawing due to inclination of the anode surface 11a.

The focus structure 13 is a structure having a function of correcting the trajectory of the electrons emitted from the electron emission part 10 and is disposed between the electron emission part 10 and the target 12 disposed on the anode surface 11a. The focus structure 13 has a window 13h. The electrons emitted from the electron emission part 10 are directed to the target 12 through the window 13h. The focus structure 13 is supplied with focus voltage Vf from the controller 2. The focus voltage Vf plays a role of controlling the amount of correction of the electron trajectory made by the focus structure 13. The focus structure 13 may be divided into two or more areas and, in this case, it is possible to adjust the focus position of an electron beam on the anode surface 11a by applying different focus voltages Vf to the respective areas.

The controller 2 is a processor that operates according to a program written in advance or an external instruction and has functions of supplying the ground potential GND to the cathode part 20, supplying the gate voltage Vg to the gate electrode 22, and supplying the focus voltage Vf to the focus structure 13. The X-ray tube 3 is activated when the supply of the gate voltage Vg to the gate electrode 22 is started under the control of the controller 2 and starts X-ray emission.

Further, the controller 2 also has a function of moving the distributed X-ray source including the plurality of X-ray tubes 3 in a predetermined direction by a predetermined distance by controlling an actuator (not illustrated) provided for the distributed X-ray source. This moving control will be described in detail later.

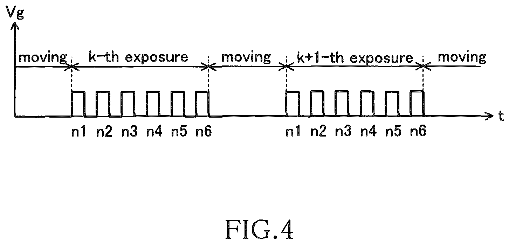

FIGs. 2A and 2B are views explaining a distributed X-ray source G according to the present embodiment. As illustrated in FIG. 2A, the distributed X-ray source G according to the present embodiment has a structure in which six X-ray tubes 3 are linearly arranged at a fixed pitch PI (e.g., 50 mm). The X-ray irradiation direction of each X-ray tube 3 is adjusted to the direction of an examination region EX of a patient in advance. Although not illustrated, an X-ray detector is disposed on the side opposite to the side on which the X-ray tubes 3 are positioned with respect to the examination region EX. The number of the X-ray detectors may be one, or the X-ray detector may be provided for each X-ray tube 3. In the example of FIGs. 2A and 2B, numbers n1 to n6 are assigned to the six X-ray tubes 3 in this order from one end side of the distributed X-ray source G, which correspond to n1 to n6 illustrated in FIG. 4 to be described later.

FIGs. 2A and 2B illustrate an example in which the examination region EX needs to be imaged twelve times at a pitch of 24/11° from 0° to 24°. On the other hand, the pitch PI of the six X-ray tubes 3 constituting the distributed X-ray source G corresponds to 48/11° as viewed from the examination region EX. The controller 2 performs imaging six times by sequentially driving the six X-ray tubes 3 constituting the distributed X-ray source G (see FIG. 2A) and then moves the entire distributed X-ray source G by a distance corresponding to PI/2, followed by imaging six times once again by sequentially driving the six X-ray tubes 3 constituting the distributed X-ray source G (see FIG. 2B). Thus, even though only six X-ray tubes 3 arranged at a pitch of 48/11° are provided, it is possible to perform the imaging of the examination region EX 12 times at a pitch of 24/11° from 0° to 24°.

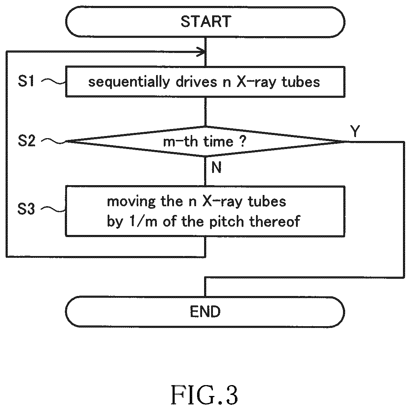

FIG. 3 is a flow diagram illustrating a control method for the X-ray imaging apparatus 1 according to the present embodiment. The control method illustrated in FIG. 3 is executed by the controller 2 illustrated in FIG. 1A. FIG. 4 is a view explaining the control method for the X-ray imaging apparatus 1 according to the present embodiment. In FIG. 4, the horizontal axis represents time t, and the vertical axis collectively represents the gate voltages Vg to be supplied to the gate electrodes 22 of the respective X-ray tubes 3. Hereinafter, with reference to FIGs. 3 and 4, the processing performed by the controller 2 will be described in more detail . For generalization, it is assumed that the number of the X-ray tubes 3 constituting the distributed X-ray source G is n and that imaging is performed n × m times. In FIG. 4, n is set to 6.

As illustrated in FIG. 3, the controller 2 sequentially drives n X-ray tubes 3 constituting the distributed X-ray source G (step S1, driving step). Specifically, as illustrated in FIG. 4, the controller 2 sequentially activates the gate voltages Vg to be supplied to the gate electrodes 22 of the respective X-ray tubes 3. In the present embodiment, the X-ray tube 3 to be activated is selected in the arrangement order (ascending order from n1 to n6) of the X-ray tubes 3 in the distributed X-ray source G. Each X-ray tube 3 is activated when the gate electrode 22 is tuned ON while the gate voltage Vg corresponding thereto is in an active state. As illustrated in FIG. 4, the gate voltages Vg are preferably supplied to the respective X-ray tubes 3 at equal intervals and so as not to allow the simultaneous activation of two or more gate voltages Vg.

Subsequently, the controller 2 determines whether or not step S1 is at the m-th time (step S2). When determining that step S1 is at the m-th time, the controller 2 ends this processing flow. On the other hand, when determining that step S1 is not at the m-th time, the controller 2 moves the n X-ray tubes 3 constituting the distributed X-ray source G by a distance corresponding to 1/m of the pitch of the X-ray tubes 3 (step S3, movement step). After that, the controller 2 returns to step S1 and executes the driving step again.

Thus, the controller 2 executes the driving step (step S1) m times with the movement step intervened therebetween. Further, in one movement step, the controller 2 moves the plurality of X-ray tubes 3 by a distance corresponding to 1/m of the pitch of the X-ray tubes 3.

As described above, according to the control method for the X-ray imaging apparatus 1 of the present embodiment, it is enough to prepare the X-ray tubes 3 of half or less than the number of required imaging positions and, in addition, imaging time can be reduced as compared to a case when imaging is performed while moving one X-ray tube 3, so that it is possible to reduce the number of the X-ray tubes 3 constituting the distributed X-ray source G.

It is apparent that the present invention is not limited to the above embodiments, but may be modified and changed without departing from the scope and spirit of the invention.

FIG. 5 is a view explaining the control method for the X-ray imaging apparatus according to a first modification of the embodiment of the present invention. The example illustrated in FIG. 5 differs from the above embodiment in the driving order of the plurality of X-ray tubes 3 in step S1 of FIG. 3. Specifically, the controller 2 according to the present modification sequentially selects every second X-ray tube 3 (in the order of n1, n3, and n5) from among all the X-ray tubes 3 from one end side of the distributed X-ray source G and activates the corresponding gate voltage Vg and, thereafter, sequentially selects the remaining X-ray tubes 3 (in the order of n2, n4, and n6) and activates the corresponding gate voltages Vg. By performing such interlace scanning, it is possible to suppress deterioration in image quality due to motion of a patient. That is, even if blurs occur in the last two images (n4 and n6) at the (k + 1)-th exposure, it is possible to correct the image blurs to some extent by complementing information from peripheral positions of the interlace scanning.

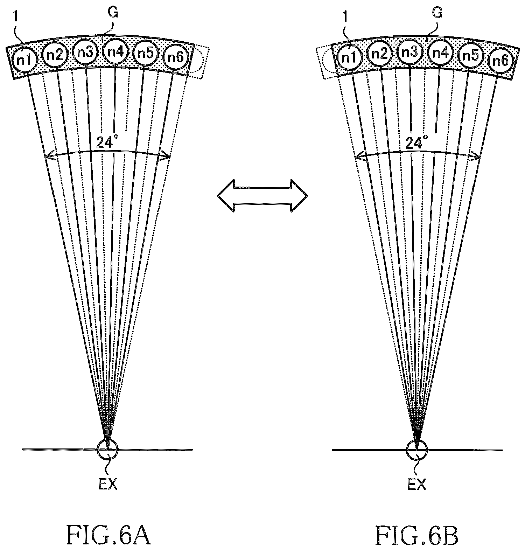

FIG. 6 is a view explaining the distributed X-ray source G according to a second modification of the embodiment of the present invention. The distributed X-ray source G illustrated in FIG. 6 differs from the distributed X-ray source G according to the above embodiment in that the X-ray tubes 3 are arranged on a circular arc centered on the examination region EX such that the distances between the examination region EX and the x-ray tubes 3 are equal. Further, the controller 2 according to the present modification is configured to execute step S3 of FIG. 3 by rotational motion centered on the examination region EX. With this configuration, the distances between the examination region EX and the x-ray tubes 3 are equal before and after the movement step, making it possible to perform imaging with higher accuracy.

- 1: cold cathode X-ray tube
- 2: controller
- 10: electron emission part
- 11: anode part
- 11a: anode surface
- 12: target
- 13: focus structure
- 13h: window
- 14: hydrogen generation part
- 15: housing
- 20: cathode part
- 21: electron emission element
- 22: gate electrode
- 22h: opening
- P: power supply
- T: transistor

## Claims

1. A control method for an X-ray imaging apparatus provided with a plurality of X-ray tubes arranged at fixed relative positions, the method comprising:
a driving step of sequentially driving the plurality of X-ray tubes; and
a movement step of moving the plurality of X-ray tubes,
wherein the driving step is executed again after execution of the movement step.

2. A control step for an X-ray imaging apparatus,
wherein the plurality of X-ray tubes are arranged at a fixed pitch,
wherein the driving step is executed m times with the movement step intervened therebetween, and,
wherein, in the movement step, the plurality of X-ray tubes are moved by a distance corresponding to 1/m of the pitch of the X-ray tubes for each time.
